(19) Europäisches Patentamt
European Patent Office
Office européen des brevets

(11) Veröffentlichungsnummer: **0 281 908 B1**

(12) # EUROPÄISCHE PATENTSCHRIFT

(45) Veröffentlichungstag der Patentschrift: **24.04.91 Patentblatt 91/17**

(51) Int. Cl.[5]: **A01N 37/18,** A01N 37/20, A01N 43/36, A01N 43/40, A01N 43/44, A01N 43/46, C07D 211/22

(21) Anmeldenummer: **88103119.9**

(22) Anmeldetag: **02.03.88**

(54) **Mittel zur Insekten- und Milbenabwehr.**

Die Akte enthält technische Angaben, die nach dem Eingang der Anmeldung eingereicht wurden und die nicht in dieser Patentschrift enthalten sind.

(30) Priorität: **12.03.87 DE 3708033**

(43) Veröffentlichungstag der Anmeldung: **14.09.88 Patentblatt 88/37**

(45) Bekanntmachung des Hinweises auf die Patenterteilung: **24.04.91 Patentblatt 91/17**

(84) Benannte Vertragsstaaten: **AT BE CH DE ES FR GB GR IT LI LU NL SE**

(56) Entgegenhaltungen:
**CH-A- 646 581**
**DE-A- 3 106 877**
**GB-A- 1 604 855**
**US-A- 3 178 439**

(56) Entgegenhaltungen:
**US-A- 4 298 612**
**Chemical Abstracts, Band 83, Nr. 25, 22 Dezember 1975, Columbus, Ohio, US K. Mizuno " Polyhydroxy amides" Seite 350, Spalte 2, Zusammenfassung-Nr. 205 791u**

(73) Patentinhaber: **BAYER AG**
**W-5090 Leverkusen 1 Bayerwerk (DE)**

(72) Erfinder: **Krüger, Bernd-Wieland, Dr.**
**Sillerstrasse 49**
**W-5600 Wuppertal 11 (DE)**
Erfinder: **Sasse, Klaus, Dr.**
**Pützweg 13**
**W-5060 Bergisch-Gladbach 2 (DE)**
Erfinder: **Hoever, Franz-Peter, Dr.**
**Kunstfelder Strasse 25**
**W-5000 Koeln 80 (DE)**
Erfinder: **Nentwig, Günther, Dr.**
**Wolfskaul 2**
**W-5000 Koeln 80 (DE)**
Erfinder: **Behrenz, Wolfgang, Dr.**
**Untergruendemich 14**
**W-5063 Overath (DE)**

**EP 0 281 908 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von teilweise bekannten acylierten α,ω-Aminoalkohol-Derivaten als insekten- und milbenabwehrende Mittel.

Mittel, die Insekten und Milben abweisen (Repellents), haben die Aufgabe, schädliche oder lästige Gliederfüßler von Berührung, sowie vom Stechen und Saugen oder Beißen an für sie anlockenden Oberflächen, etwa der Haut von Tieren und Menschen abzuhalten, wenn diese zuvor mit solchen Mitteln behandelt wurden.

Als Repellents wurden bereits zahlreiche Wirkstoffe vorgeschlagen. (Vergl, z.B. K.H. Büchel in Chemie der Pflanzenschutz- und Schädlingsbekämpfungsmittel ; Herausgeber : R. Wegler, Bd. 1, Springer Verlag Berlin, Heidelberg, New York, 1970 S. 487 ff).

Besonders bekannt und seit längerer Zeit in Gebrauch sind 3-Methyl-benzoesäurediethylamid (DEET), Dimethylphthalat und 2-Ethyl-hexandiol-1,3, von denen vor allem das DEET in der Praxis eine erhebliche Bedeutung erlangt hat (siehe zB. R.K. Kocher, R.S. Dixit, C.I. Somaya ; Indian J. Med. Res. 62, 1 (1974)).

Ein erheblicher Nachteil der bekannten Repellents ist ihre zum Teil relativ kurze (nur wenige Stunden) anhaltende Dauewirkung.

Ein Teil der durch die nachfolgende Formel (I) definierten Verbindungen ist bekannt.

Entsprechende Polyhydroxyamine sind z.B. aus Chemical Abstracts 83 (25) : 205/791 u bekannt. Diese enthalten zwingend zwei freie Hydroxylgruppen und werden als Weichmacher für Amidharze eingesetzt.

Weiterhin sind N-Alkanoyl- und -Alkenoyl-hydroxyalkyl-piperidine aus US-Patent No. 3 178 439 bekannt. Weitere Piperidine sind aus Tetrahedron Suppl. 8/Teil 1 (1966) S. 113-121 bekannt.

Eine insekten- und milbenabweisende Wirkung dieser Verbindungen ist jedoch bisher nicht bekannt geworden.

Aus US-Patent No. 4 298 612 sind Insectrepellents auf Carbonsäureamid-Basis bekannt. Diese sind jedoch den erfindungsgemäß eingesetzten α,ω-Aminoalkohol-Derivaten wirkungsmäßig eindeutig unterlegen.

Aus GB-A 1 604 855 sind $C_{1-6}$-Alkanol-N-substituierte Fettsäureamide mit einer stark toxischen Wirkung gegen Ektoparasiten, insbesondere gegen Läuse und deren Eier, bekannt. Eine Repellentwirkung gegen Insekten und Milben wird hierin weder beschrieben noch nahegelegt. Es wurde nun gefunden, daß die acylierten α,ω-Aminoalkohol-Derivate der Formel (I)

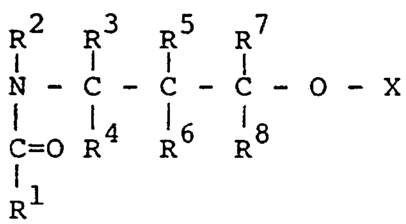

(I)

in welcher

X für Wasserstoff, $COR^{11}$ oder $R^{13}$ steht,

$R_1$ für $C_1$-$C_7$-Alkyl oder $C_3$-$C_7$-Alkenyl steht,

$R^2$, $R^{11}$, $R^{13}$ gleich oder verschieden sind und für $C_1$-$C_6$-Alkyl stehen,

$R^3$-$R^8$ gleich oder verschieden sind und für Wasserstoff oder $C_1$-$C_6$-Alkyl stehen, wobei $R^2$ und $R^3$ gemeinsam mit den Atomen, an welche sie gebunden sind, einen 5- oder 6-gliedrigen monocyclischen Ring bilden können,

eine starke insekten- und milbenabweisende Wirkung (Repellentwirkung) besitzen.

Die Repellentwirkung ist erheblich besser als die der aus dem Stand der Technik bekannten Repellents. Die erfindungsgemäßen Wirkstoffe stellen somit eine wertvolle Bereicherung der Technik dar.

Die vorliegende Erfindung betrifft somit die Verwendung acylierter α,ω-Aminoalkoholderivate der allgemeinen Formel I zur Insekten- und Milbenabwehr.

Weiterhin betrifft die Erfindung insekten- und milbenabweisende Mittel, gekennzeichnet durch den Gehalt an mindestens einem acylierten α,ω-Aminoalkoholderivat der allgemeinen Formel I.

Die erfindungsgemäßen Mittel, die mindestens ein Derivat der Formel I enthalten, können auch weitere Insektenabwehrmittel enthalten. Hier kommen alle praktisch üblichen Repellentien in Frage (vergl. z.B. K.H. Büchel in Chemie der Pflanzenschutz- und Schädlingsbekämpfungsmittel ; Herausg. : R. Wegler, Bd. 1, Springer Verlag Berlin, Heidelberg, New York, 1970, S. 487 ff).

Im Falle der Repellentkombinationen werden bevorzugt die acylierten α,ω-Aminoalkohole der allgemeinen

Formel I zusammen mit repellenten Carbonsäureamiden, 1,3-Alkandiolen und Carbonsäureestern verwendet. Im einzelnen seien genannt: 3-Methyl-benzoesäurediethylamid (DEET), 2-Ethyl-hexandiol-1,3 (Rutgers 612) und Phthalsäuredimethylester.

Die erfindungsgemäß verwendbaren acylierten α,ω-Aminoalkoholderivate sind durch die allgemeine Formel (I) charakterisiert.

Besonders bevorzugt werden Verbindungen der allgemeinen Formel (I) als Repellentien verwendet, in welcher

X für Wasserstoff oder $R^{13}$ steht,

wobei $R^{13}$ für $C_1$-$C_6$-Alkyl steht,

$R^1$ für $C_1$-$C_7$-Alkyl oder $C_3$-$C_7$-Alkenyl steht,

$R_4$ bis $R_8$ gleich oder verschieden sind und für Wasserstoff oder $C_1$-$C_6$-Alkyl stehen,

$R^2$ und $R^3$ gemeinsam mit den Atomen, an welche sie gebunden sind, einen 5- oder 6-gliedrigen monocyclischen Ring bilden.

Weiterhin werden Verbindungen bevorzugt, in denen $R^1$ für $C_1$-$C_7$-Alkyl oder $C_3$-$C_7$-Alkenyl steht, X für $COR^{11}$ oder $R^{13}$ steht, $R^2$ und $R^{11}$ gleich oder verschieden sind und für $C_1$-$C_6$-Alkyl stehen, $R^3$ bis $R^8$ gleich oder verschieden sind und für Wasserstoff oder $C_1$-$C_6$-Alkyl stehen, $R^{13}$ für $C_1$-$C_6$-Alkyl steht.

Ganz besonders bevorzugt werden die Verbindungen der allgemeinen Formel (I) als Repellentien verwendet, in denen $R^1$ für $C_1$-$C_4$-Alkyl steht,

$R^2$, $R^{11}$ und $R^{13}$ gleich oder verschieden sind und für $C_1$-$C_6$-Alkyl stehen, $R^3$ bis $R^8$ für Wasserstoff stehen und X für Wasserstoff, $COR^{11}$ oder $R^{13}$ steht, wobei $R^{11}$ und $R^{13}$ die vorgenannte Bedeutung haben.

Weiterhin werden ganz besonders bevorzugt Verbindungen der allgemeinen Formel (I) als Repellentien verwendet, in denen $R^1$ für $C_3$-$C_4$-Alkyl steht, $R^2$ und $R^3$ gemeinsam mit den Atomen, an die sie gebunden sind, einen 6-gliedrigen Ring bilden, $R^4$ bis $R^8$ für Wasserstoff steht und X für Wasserstoff und $R^{13}$ steht, wobei $R^{13}$ für $C_1$-$C_4$-Alkyl steht.

Die Verbindungen der allgemeinen Formel (I) sind entweder bekannt oder können nach bekannten Methoden und Verfahren hergestellt werden (vergl. z.B. Cesare Ferri, Reaktionen der organischen Synthese, Georg Thieme Verlag Stuttgart, 1978, S. 223 und S. 450).

Man erhält demgemäß die Verbindungen der Formel (I), wenn man die an sich bekannten oder nach bekannten Verfahren herstellbaren α,ω-Aminoalkohole (vergl. z.B. Cesare Ferri, Reaktionen der org. Synthese, Georg Thieme Verlag Stuttgart, 1978, S. 211 ff bzw. 496-497) der Formel (II)

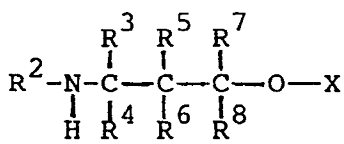

(II)

worin $R^2$ bis $R^8$ die unter Formel (I) angegebene Bedeutung haben, zunächst mit an sich bekannten Carbonsäurechloriden der Formel (III)

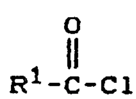

(III),

wobei

$R^1$ die unter Formel (I) angegebene Bedeutung hat,

gegebenenfalls in Gegenwart eines Säureakzeptors, wie z.B. Triethylamin oder Kaliumcarbonat und gegebenenfalls unter Verwendung eines Verdünnungsmittels, wie z.B. Toluol, $CH_2Cl_2$, Tetrahydrofuran oder Acetonitril, bei Temperaturen zwischen −40 und 110°C, umsetzt.

Für die Herstellung von Verbindungen der allgemeinen Formel (I), in denen X verschieden von Wasserstoff ist, erfolgt dann in einem zweiten Reaktionsschritt, gegebenenfalls nach Isolierung des Zwischenproduktes mit freier OH-Gruppe, die weitere Acylierung/Alkylierung mit an sich bekannten Carbonsäurechloriden der Formel (IV)

$$R^{11}COCl \quad (IV),$$

oder die Umsetzung mit Alkylhalogeniden der Formel (VI)

$R^{13}$-Y (VI),

zur Herstellung von Verbindungen der Formel (I) mit X = $R^{13}$; wobei in den Formeln (IV), und (VI) Y für Chlor, Brom oder Iod, vorzugsweise für Brom oder Iod steht und $R^{11}$ und $R^{13}$ die obengenannte Bedeutung haben, gegebenenfalls in Gegenwart eines Säureakzeptors wie z.B. Triethylamin oder Kaliumcarbonat oder einer Base wie Natriumhydrid, gegebenenfalls unter Verwendung eines Verdünnungsmittels, wie z.B. Toluol, Tetrahydrofuran oder Acetonitril, bei Temperaturen zwischen 0 und 110°C umsetzt. Die Aufarbeitung erfolgt nach üblichen Methoden, beispielsweise durch Extraktion der Produkte mit Methylenchorid oder Toluol aus der mit Wasser verdünnten Reaktionsmischung, Waschen der organischen Phase mit Wasser, Trocknen und Destillieren oder sogenanntes "Andestillieren", d.h. durch längeres Erhitzen unter vermindertem Druck auf mäßig erhöhte Temperaturen, um von den letzten flüchtigen Bestandteilen zu befreien.

Eine weitere Reinigung kann durch Chromatographie an Kieselgel mit z.B. Hexan : Aceton = 7 : 3 als Laufmittel erfolgen.

Zur Charakterisierung der Verbindungen dienen Brechungsindex, Schmelzpunkt, Rf-Wert oder Siedepunkt.

Die vorliegende Erfindung betrifft auch neue acylierte α,ω-Aminoalkohol-Derivate der Formel

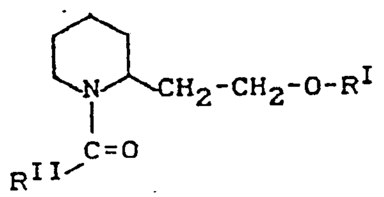

(Ia)

worin

$R^I$ für Wasserstoff, $COR^{11}$ oder $R^{13}$ steht, wobei $R^{11}$ und $R^{13}$ gleich oder verschieden sind und für gegebenenfalls substituierte Alkyl- oder Alkenylreste stehen

und

$R^{II}$ für $C_2$-$C_6$-Alkyl oder für $C_3$-$C_6$-Alkenyl steht.

Bevorzugte Verbindungen sind solche der Formel (Ia) in welcher

$R^I$ für Wasserstoff oder den Rest $R^{13}$ steht, wobei $R^{13}$ für gegebenenfalls substituiertes Alkyl oder Alkenyl steht und

$R^{II}$ $C_2$-$C_6$-Alkyl oder $C_3$-$C_6$-Alkenyl bedeutet.

Man erhält die acylierten α,ω-Aminoalkohol-Derivate der Formel

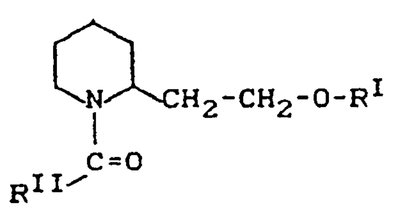

(Ia)

worin

$R^I$ für Wasserstoff, $COR^{11}$, $R^{13}$ steht, wobei $R^{11}$ und $R^{13}$ gleich oder verschieden sind und für gegebenenfalls substituierte Alkyl- oder Alkenylreste stehen

und

$R^{II}$ für $C_2$-$C_6$-Alkyl oder für $C_3$-$C_6$-Alkenyl steht, wenn man den α,ω-Aminoalkohol der Formel

mit einem Carbonsäurechlorid der Formel

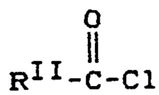

(IIIa)

wobei $R^{II}$ die oben angegebene Bedeutung besitzt,
gegebenenfalls in Gegenwart eines Säureakzeptors wie z.B. Triethylamin oder Kaliumcarbonat und gegebenenfalls unter Verwendung eines organischen Verdünnungsmittels wie z.B. Toluol, Methylenchlorid, Tetrahydrofuran oder Acetonitril bei Temperaturen zwischen–40 und 110°C umsetzt, die Verbindungen der Formel

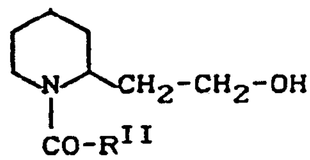

(Ib)

gegebenenfalls isoliert und gegebenenfalls weiter in einem zweiten Reaktionsschritt, die weitere Acylierung/Alkylierung mit an sich bekannten Carbonsäurehalogeniden (insbesondere Carbonsäurechloriden) der Formel

$R^{11}COCl$ (IV)

zum Erhalt von Verbindungen der Formel Ia mit $R^I = COR^{11}$ oder mit Alkylhalogeniden der Formel

$R^{13}$-Y (IV)

zum Erhalt von Verbindunge der Formel Ia mit $R^I = R^{13}$
wobei

$R^{11}$ und $R^{13}$ die oben angegebene Bedeutung haben

und

Y für Chlor, Brom oder Iod, vorzugsweise für Brom oder Iod steht,

gegebenenfalls in Gegenwart eines Säureakzeptors wie z.B. Triethylamin oder Kaliumcarbonat oder einer Base wie Natriumhydrid, gegebenenfalls unter Verwendung eines organischen Verdünnungsmittels, wie z.B. Toluol, Tetrahydrofuran oder Acetonitril vorzugsweise bei Temperaturen zwischen 0 und 110°C umsetzt.

Die Aufarbeitung der erfindungsgemäßen Verbindungen der Formel (Ia) geschieht in analoger Weise, wie weiter vorne, bei der Herstellung der Verbindungen der Formel (I) beschrieben.

Die Wirkung der Repellentien der allgemeinen Formel (I) hält lange an.

Sie können daher mit gutem Erfolg zur Abwehr von schädlichen oder lästigen, saugenden und beißenden Insekten und Milben verwendet werden.

Zu den saugenden Insekten gehören im wesentlichen die Stechmücken (z.B. Aedes-, Culex- und Anopheles-Arten), Schmetterlingsmücken (Phlebotomen), Gnitzen (Culicoides-Arten), Kriebelmücken (Simulium-Arten), Stechfliegen (z.B. Stomoxys calcitrans), Tsetse-Fliegen (Glossina-Arten), Bremsen (Tabanus-, Haematopota- und Chrysops-Arten), Stubenfliegen (z.B. Musca domestica und Fannia canicularis), Fleischfliegen (z.B. Sarcophaga carnaria), Myiasis erzeugende Fliegen (z.B. Lucilia cuprina, Chrysomyia chloropyga, Hypoderma bovis, Hypoderma lineatum, Dermatobia hominis, Oestrus ovis, Gasterophilus intestinalis, Cochliomyia hominovorax), Wanzen (z.B. Cimex lectularius, Rhodnius prolixus, Triatoma infestans), Läuse (z.B. Pediculus humanus, Haematopinus suis, Damalina oris), Lausfliegen (z.B. Melaphagus orinus), Flöhe (z.B. Pulex irritans, Cthenocephalides canis, Xenopsylla cheopis) und Sandflöhe (z.B. Dermatophilus penetrans).

Zu den beißenden Insekten gehören im wesentlichen Schaben (z.B. Blatella germanica, Periplaneta americana, Blatta orientalis, Supella supellectilium), Käfer (z.B. Sitophilus granarius, Tenebrio molitor, Dermestes lardarius, Stegobium paniceum, Anobium puntactum, Hylotrupes bajulus), Termiten (z.B. Reticulitermes lucifugus) und Ameisen (z.B. Lasius niger).

Zu den Milben gehören Zecken (z.B. Ornithodorus moubata, Ixodes ricinus, Boophilus microplus, Amblyomma hebreum) und Milben in engerem Sinne (z.B. Sarcoptes scabiei, Dermanyssus gallinae).

Die erfindungsgemäßen Wirkstoffe, die unverdünnt oder vorzugsweise verdünnt eingesetzt werden können, lassen sich in die für Repellents üblichen Formulierungen überführen. Sie lassen sich in allen in der Kosmetik üblichen Darreichungsformen einsetzen, beispielsweise in Form von Lösungen, Emulsionen, Gelen, Salben, Pasten, Cremes, Pulvern, Stiften, Sprays oder Aerosolen aus Sprühdosen.

Für die Anwendung im nichtkosmetischen Bereich lassen sich die Wirkstoffe z.B. in Granulate, Ölsprühmittel oder Slow-Release-Formulierungen einarbeiten.

Die Zubereitungen werden in bekannter Weise durch Vermischen oder Verdünnen der erfindungsgemäßen Wirkstoffe mit Lösungsmitteln (z.B. Xylol, Chlorbenzole, Paraffine, Methanol, Ethanol, Isopropanol, Wasser), Trägerstoffe (z.B. Kaoline, Tonerden, Talkum, Kreide, hochdisperse Kieselsäure, Silikate), Emulgiermitteln (z.B. Polyoxyethylen-Fettsäure-Ester, Polyoxyethylen-Fettalkohol-Ether, Alkylsulfonate, Arylsulfonate) und Dispergiermitteln (z.B. Lignin-Sulfitablaugen, Methylcellulose) hergestellt.

Die erfindungsgemäßen Wirkstoffe können in den Formulierungen miteinander gemischt oder auch in Mischungen mit anderen bekannten Wirkstoffen (z.B. Sonnenschutzmittel) eingesetzt werden. Die Zubereitungen enthalten im allgemeinen zwischen 0,1 und 95 Gew.-% Wirkstoff, vorzugsweise zwischen 0,5 und 90%.

Zum Schutz gegen blutsaugende Insekten oder Milben werden die erfindungsgemäßen Wirkstoffe entweder auf die menschliche oder tierische Haut aufgebracht oder Kleidungsstücke und andere Gegenstände damit behandelt.

Auch als Zusatz von Imprägniermitteln für beispielsweise Textilbahnen, Kleidungsstücke, Verpackungsmaterialien, sowie als Zusatz zu Polier-, Putz- und Fensterreinigungsmitteln sind die erfindungsgemäßen Wirkstoffe geeignet.

Die folgenden Beispiele für die Zubereitungen und die Verwendung der erfindungsgemäßen Wirkstoffe dienen der weiteren Erläuterung der Erfindung.

## Beispiel 1

Ein Repellentmittel in Form einer Lotion zur Anwendung auf der Haut wird hergestellt durch Vermischen von 30 Teilen eines der erfindungsgemäßen Wirkstoffe, 1,5 Teilen Parfüm und 68,5 Teilen Isopropanol. Isopropanol kann durch Ethanol ersetzt werden.

## Beispiel 2

Ein Repellentmittel in Form eines Aerosols zum Aufsprühen auf die Haut wird hergestellt, indem man 50% Wirkstofflösung, bestehend aus 30 Teilen eines der erfindungsgemäßen Wirkstoffe, 1,5 Teilen Parfüm, 68,5 Teilen Isopropanol, mit 50% Frigen 11/12 (= halogenierter Kohlenwasserstoff als Treibgas) als Sprühdosenpräparation formuliert.

## Beispiel 3

Eine andere Sprühdose setzt sich aus 40% Wirkstofflösung, bestehend aus 20 Teilen eines der erfindungsgemäßen Wirkstoffe, 1 Teil Parfüm, 79 Teilen Isopropanol und 60% Propan/Butan (Verhältnis 15 : 85) zusammen.

Es wurden individuelle Formulierungen entsprechend den Beispielen 1, 2 und 3 unter Einsatz folgender Wirkstoffe hergestellt : Verbindungen gemäß Herstellungsbeispielen Nr. 1, 2, 3, 6, 13.

Die folgenden Beispiele der biologischen Wirkung zeigen die Überlegenheit der erfindungsgemäßen Substanzen gegenüber dem Stand der Technik (Diethyltoluamid = DEET) :

## Beispiel A

Repellenttest am Meerschweinchen

| | |
|---|---|
| Testtier : | Aedes aegypti (Imagines) |
| Zahl der Testtiere : | ca. 5000 |
| Lösungsmittel : | Ethanol (99,8%) |

3 Gewichtsteile Wirkstoff werden in 100 Volumenteilen Lösungsmittel aufgenommen.

Ein Meerschweinchen, dessen Rücken in einem Bereich von 50 $cm^2$ rasiert worden ist, wird in einem engen Käfig (Box) so fixiert, daß nur die rasierte Fläche den Mücken zugänglich ist. Nach Behandeln der Fläche mit 0,4 ml Wirkstofflösung wird das Meerschweinchen nach Verdunsten des Lösungsmittels samt Box in einen 60

× 60 × 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen. Anschließend wird dieses herausgenommen und der Test nach einer Stunde wiederholt. Der Versuch wird maximal 14 Stunden lang durchgeführt oder so lange, bis die Wirkung abbricht.

Bei diesem Test zeigen z.B. die folgenden Verbindungen der Herstellungsbeispiele überlegene Wirkung gegenüber dem Stand der Technik (Diethyltoluamid = DEET) : (1), (2), (3), (6), (13), (26).

Tabelle A

Repellenttest am Meerschweinchen

| Präparat | | Anzahl der Einstiche nach: $0^h-6^h$ | $7^h-14^h$ |
|---|---|---|---|
| Erfindungsgemäß: Hst.-Bsp. Nr. 1 | 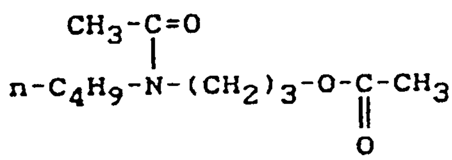 | 0,1 | 6,3 |
| Hst.-Bsp. Nr. 6 | 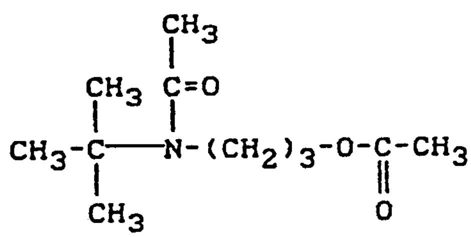 | 1,9 | 3,6 |
| Hst.-Bsp. Nr. 2 | 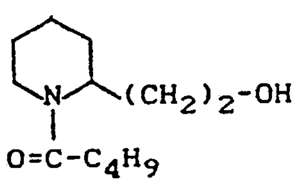 | 0,1 | 0,6 |
| Hst.-Bsp. Nr. 13 | 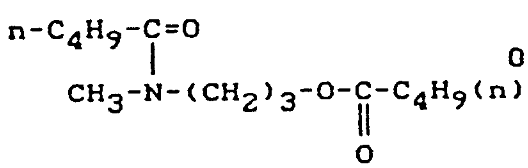 | 0,3 | 1,6 |
| Hst.-Bsp. Nr. 3 | 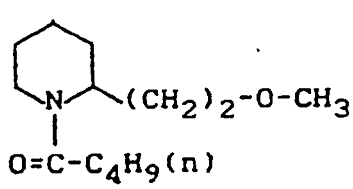 | 0,1 | 3,5 |

Anmerkung: "Hst.-Bsp." bedeutet "Herstellungsbeispiel"

Tabelle A (Fortsetzung)

Repellenttest am Meerschweinchen

| Präparat | | Anzahl der Einstiche nach: $0^h$-$6^h$ | $7^h$-$14^h$ |
|---|---|---|---|
| Hst.-Bsp. Nr. 26 | 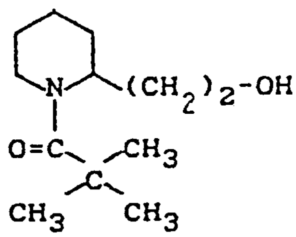 | 0,1 | 2,4 |
| Bekannt: DEET | 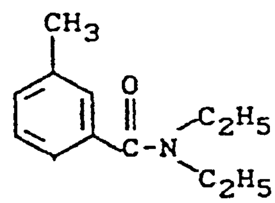 | 2,4 | 11,6 |

Beispiel B

Repellenttest am Meerschweinchen

Testtiere : Culex pipiens fatigans

Zahl der Testtiere : ca. 1000

Lösungsmittel : Ethanol (99,8%)

3 Gewichtsteile Wirkstoff werden in 100 Volumenteilen Lösungsmittel aufgenommen.

Ein Meerschweinchen, dessen Rücken in einem Bereich von 50 $cm^2$ rasiert worden ist, wird in einem engen Käfig (Box) so fixiert, daß nur die rasierte Fläche den Mücken zugänglich ist. Nach Behandeln der Fläche mit 0,4 ml Wirkstofflösung wird das Meerschweinchen nach Verdunsten des Lösungsmittels samt Box in einem 60 × 60 × 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 10 Stunden lang durchgeführt oder so lange, bis die Wirkung abbricht. Bei diesem Test zeigen z.B. die folgenden Verbindungen der Herstellungsbeispiele überlegene Wirkung gegenüber dem Stand der Technik (Diethyltoluamid = DEET) : (3), (8), (15), (28).

<u>Tabelle B</u>

Repellenttest am Meerschweinchen

| Präparat | | Anzahl der Einstiche nach: $0^h$-$6^h$ | $7^h$-$10^h$ |
|---|---|---|---|
| Erfindungsgemäß:<br>Hst.-Bsp. Nr. 6 | 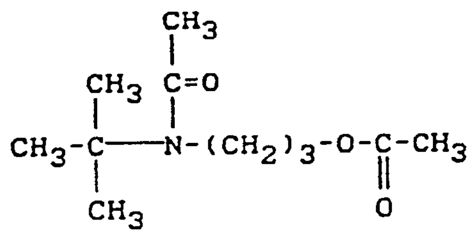 | 0,1 | 3,1 |
| Hst.-Bsp. Nr. 13 | 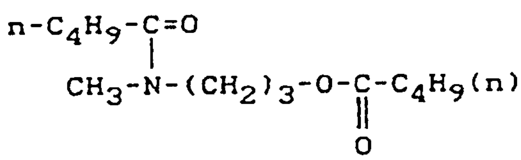 | 0,1 | 2,2 |
| Hst.-Bsp. Nr. 3 | 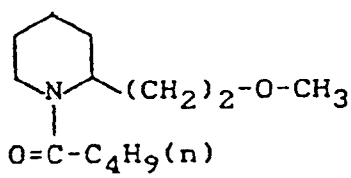 | 0 | 0,6 |

T a b e l l e B (Fortsetzung)

Repellenttest am Meerschweinchen

| Präparat | | Anzahl der Einstiche nach: $0^h$-$6^h$ | $7^h$-$10^h$ |
|---|---|---|---|
| Hst.-Bsp. Nr. 26 | 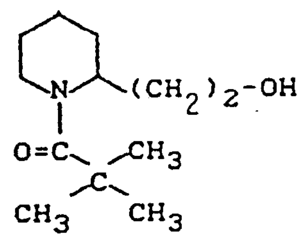 | 0 | 0,2 |
| Bekannt: DEET | 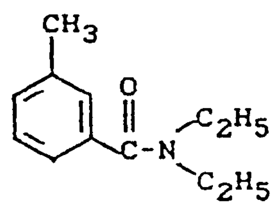 | 0,1 | 4,9 |

Anmerkung: "Hst.-Bsp." bedeutet "Herstellungsbeispiel"

## Herstellungsbeispiele

### Herstellungsbeispiel 1

N,O-Bis-acetyl-N-butyl-aminopropanol-1,3

40 g (0,3 Mol) N-Butyl-aminopropanol-1,3 und 100 ml Triethylamin (0,72 Mol) werden in 1 Liter Tetrahydrofuran gelöst und bei 20°C mit 50 ml Acetylchlorid (0,7 Mol) versetzt. Man erwärmt einen Tag unter Rückfluß und filtriert dann den Feststoff ab. Es wird Methylenchlorid zugegeben und die organische Phase mit Wasser gewaschen. Anschließend wird die organische Phase mit Magnesiumsulfat getrocknet, das Lösungsmittel am Rotationsverdampfer abdestilliert und im Kugelrohrofen ($Kp_{0,6}$ 135-140°C) destilliert. Zur weiteren Reinigung wird die Substanz über 1 kg Kieselgel (Laufmittel : Cyclohexan : Aceton = 7 : 3) chromatographiert.

Ausbeute : 52,1 g = 81% der Theorie

### Herstellungsbeispiel 2

1-Pentanoyl-2-(2-hydroxyethyl)-piperidin

65 g (0,5 Mol) 2-(2-Hydroxyethyl)-piperidin und 90 ml Triethylamin (0,64 Mol) werden in 1 Liter Tetrahydrofuran gelöst und bei–20°C mit 80 ml (0,67 Mol) Valeriansäurechlorid versetzt. Man erwärmt einen Tag auf 20°C und entfernt dann das Lösungsmittel weitgehend am Rotationsverdampfer ; nimmt mit Methylenchlorid auf, wäscht mit 1N NaOH-Lösung, trocknet die organische Phase und entfernt das Lösungsmittel destallativ am Rotationsverdampfer. Um Verunreinigungen bis-acylierter Verbindung zu entfernen, wird in 200 ml Ethanol aufgenommen und eine Stunde bei 50°C mit 200 ml 1N Natronlauge erwärmt. Es wird erneut einrotiert, mit

$CH_2Cl_2/H_2O$ extrahiert, die organische Phase getrocknet, einrotiert und im Kugelrohrofen destilliert ($Kp_{0,2}$ = 165°C).

Ausbeute : 65,4 = 61% der Theorie.

Herstellungsbeispiel 3

1-Pentanoyl-2-(2-methoxethyl)-piperidin

32 g (0,15 Mol) 1-Pentanoyl-2-(2-hydroxyethyl)-piperidin werden in 300 ml Tetrahydrofuran gelöst und bei 20°C mit 5,9 g (0,197 Mol) Natriumhydrid (80%ig in Paraffin) versetzt. Man erwärmt 4 Stunden unter Rückfluß und gibt dann 20 ml Methyliodid (0,32 Mol) zur Reaktionsmischung.

Anschließend wird 8 Stunden unter Rückfluß erwärmt, dann bei 20°C mit 100 ml Ammoniumchloridlösung versetzt, mit Methylenchlorid extrahiert, die organische Phase mit Magnesiumsulfat getrocknet und einrotiert. Nach chromatographischer Reinigung (Laufmittel $CH_2Cl_2$ : i-$C_3H_7OH$ = 1 : 1 ; Kieselgel) wird einrotiert und im Kugelrohrofen destilliert ($Kp_{0,2}$ : 160°C).

Ausbeute : 27,7 g = 81% der Theorie.

In Analogie zu den vorgenannten Herstellungsbeispielen 1 bis 3 wurden die nachfolgend tabellarisch aufgeführten weiteren Herstellungsbeispiele synthetisiert.

Allgemeine Formel:

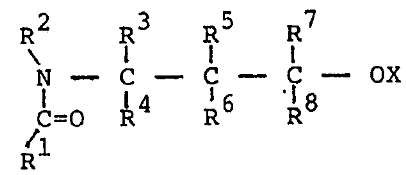

(I)

| Hst.-Bsp. Nr. | $R^1$ | $R^2$ | $R^3$ | $R^4$ | $R^5$ | $R^6$ | $R^7$ | $R^8$ | X | phys. Daten ($n_D^{20}$) |
|---|---|---|---|---|---|---|---|---|---|---|
| 4 | $CH_3$ | $n-C_4H_9$ | H | H | H | H | H | H | $COCH_3$ | 1,4565 |
| 5 | $CH_3$ | $C_2H_5$ | H | H | H | H | H | H | $COCH_3$ | 1,4532 |
| 6 | $CH_3$ | $t-C_4H_9$ | H | H | H | H | H | H | $COCH_3$ | 1,4565 |
| 7 | $CH_3$ | $n-C_3H_7$ | H | H | H | H | H | H | $COCH_3$ | 1,4540 |
| 8 | $CH_3$ | $CH_3$ | H | H | H | H | H | H | $COCH_3$ | 1,4530 |
| 9 | $CH_3$ | $CH_3$ | H | H | H | H | H | H | H | 1,4660 |
| 10 | $n-C_4H_9$ | $-(CH_2)_4-$ | | H | H | H | H | H | $COCH_3$ | 1,4770 |
| 11 | $CH_3$ | $-(CH_2)_4-$ | | H | H | H | H | H | $COCH_3$ | 1,4780 |
| 12 | $CH_3$ | $i-C_3H_7$ | H | H | H | H | H | H | $COCH_3$ | 1,4549 |
| 13 | $n-C_4H_9$ | $CH_3$ | H | H | H | H | H | H | $CO-C_4H_9-(n)$ | 1,4526 |
| 14 | $n-C_4H_9$ | $n-C_4H_9$ | H | H | H | H | H | H | H | 1,4655 |
| 15 | $n-C_5H_{11}$ | $n-C_4H_9$ | H | H | H | H | H | H | H | 1,4649 |
| 16 | $C_2H_5$ | $-(CH_2)_4-$ | | H | H | H | H | H | H | 1,4960 |
| 17 | $CH_3$ | $-(CH_2)_4-$ | | H | H | H | H | H | H | 1,4990 |

| Hst.-Bsp. Nr. | $R^1$ | $R^2$ | $R^3$ | $R^4$ | $R^5$ | $R^6$ | $R^7$ | $R^8$ | X | phys. Daten ($n_D^{20}$) |
|---|---|---|---|---|---|---|---|---|---|---|
| 18 | $n-C_3H_7$ | $-(CH_2)_4-$ | | H | H | H | H | H | H | 1,4905 |
| 19 | $n-C_4H_9$ | $CH_3$ | H | H | H | H | H | H | $CH_3$ | 1,4510 |
| 20 | $n-C_5H_{11}$ | $-(CH_2)_4-$ | | H | H | H | H | H | H | 1,4995 |
| 21 | $n-C_4H_9$ | $-(CH_2)_4-$ | | H | H | H | H | H | $C_2H_5$ | 1,4730 |
| 22 | $n-C_4H_9$ | $-(CH_2)_4-$ | | H | H | H | H | H | $n-C_3H_7$ | 1,4711 |
| 23 | $n-C_4H_9$ | $-(CH_2)_4-$ | | H | H | H | H | H | $n-C_4H_9$ | 1,4713 |
| 24 | $n-C_4H_9$ | $-(CH_2)_3-$ | | H | H | H | H | H | H | 1,4854 |
| 25 | $CH_3$ | 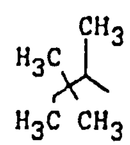 | H | H | H | H | H | H | $COCH_3$ | 1,4625 |
| 26 | $t-C_4H_9$ | $-(CH_2)_4-$ | | H | H | H | H | H | H | 1,4873 |
| 27 | $CH(C_2H_5)C_4H_9$ | $-(CH_2)_4-$ | | H | H | H | H | H | H | 1,4852 |
| 28 | $CH=CH-C_2H_5$ | $-(CH_2)_4-$ | | H | H | H | H | H | H | 1,5159 |
| 29 | $C=CH-CH_3$<br>$CH_3$ | $-(CH_2)_4-$ | | H | H | H | H | H | H | 1,5034 |
| 30 | $CH=CH-CH_3$ | $-(CH_2)_4-$ | | H | H | H | H | H | H | 1,5200 |

| Hst.-Bsp. Nr. | $R^1$ | $R^2$ | $R^3$ | $R^4$ | $R^5$ | $R^6$ | $R^7$ | $R^8$ | X | phys. Daten |
|---|---|---|---|---|---|---|---|---|---|---|
| 31 | $t-C_4H_9$ | $-(CH_2)_4-$ | | H | H | H | H | H | H | (-)-Form: $[\alpha]_{365} = -127,1^0$ (C=1,5;THF) |
| 32 | $t-C_4H_9$ | $-(CH_2)_4$ | | H | H | H | H | H | H | (+)-Form: $[\alpha]_{365} = 125,0^0$ (C = 1,5;THF) |
| 33 | $t-C_4H_9$ | $-(CH_2)_4-$ | | H | H | H | H | H | $COC_4H_9-t$ | 1,4700 ($n_D^{20}$) |
| 34 | $C(CH_3)_2C_2H_5$ | $-(CH_2)_4-$ | | H | H | H | H | H | H | 1,4906 ($n_D^{20}$) |
| 35 | $C_4H_9$ | $-(CH_2)_3-$ | | H | H | H | H | H | $COCH_3$ | 1,4747 ($n_D^{20}$) |
| 36 | $CH_3$ | $CH_2-CH=CH-CH_3$ | H | H | H | H | H | H | $COCH_3$ | |
| 37 | $CH_3$ | $CH_2-CH=CH-CH_3$ | H | H | H | H | H | H | H | |

THF = Tetrahydrofuran

## Ansprüche

1. Mittel zur Insekten- und Milbenabwehr, gekennzeichnet durch einen Gehalt an mindestens einem acylierten α,ω-Aminoalkohol-Derivat der Formel (I)

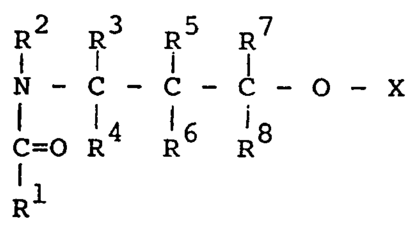

(I)

in welcher

X für Wasserstoff, $COR^{11}$ oder $R^{13}$ steht,

$R_1$ für $C_1$-$C_7$-Alkyl oder $C_3$-$C_7$-Alkenyl steht,

$R^2$, $R^{11}$, $R^{13}$ gleich oder verschieden sind und für $C_1$-$C_6$-Alkyl stehen,

$R^3$-$R^8$ gleich oder verschieden sind und für Wasserstoff oder $C_1$-$C_6$-Alkyl stehen, wobei $R^2$ und $R^3$ gemeinsam mit den Atomen, an welche sie gebunden sind, einen 5- oder 6-gliedrigen monocyclischen Ring bilden können.

2. Verfahren zur Herstellung von Mitteln zur Insekten- und Milbenabwehr, dadurch gekennzeichnet, daß man acylierte α,ω-Aminoalkohol-Derivate gemäß Formel (I) mit Streckmitteln und/oder oberflächenaktiven Mitteln vermischt.

3. Acylierte α,ω-Aminoalkohol-Derivate der Formel

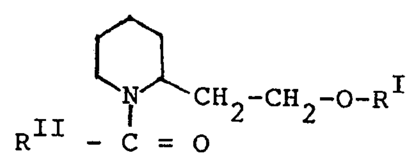

(Ia)

worin

$R^I$ für Wasserstoff, $COR^{11}$, $R^{13}$ steht, wobei $R^{11}$ und $R^{13}$ gleich oder verschieden sind und für gegebenenfalls substituierte Alkyl- oder Alkenylreste stehen und

$R^{II}$ für $C_2$-$C_6$-Alkyl oder für $C_3$-$C_6$-Alkenyl steht.

4. Acylierte α-ω-Aminoalkohol-Derivate der Formel (Ia) gemäß Anspruch 6, dadurch gekennzeichnet, daß $R^I$ für Wasserstoff oder den Rest $R^{13}$ steht, wobei $R^{13}$ für gegebenenfalls substituiertes Alkyl oder Alkenyl steht und

$R^{II}$ $C_2$-$C_6$-Alkyl oder $C_3$-$C_6$-Alkenyl bedeutet.

5. Verfahren zur Herstellung von acylierten α,ω-Aminoalkohol-Derivaten der Formel

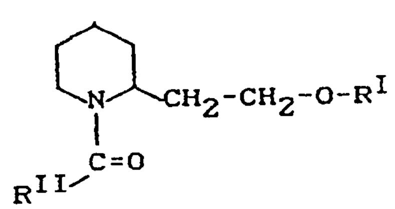

(Ia)

worin

$R^I$ für Wasserstoff, $COR^{11}$, $R^{13}$ steht, wobei $R^{11}$ und $R^{13}$ gleich oder verschieden sind und für gegebenenfalls substituierte Alkyl- oder Alkenylreste stehen

und

$R^{II}$ für $C_2$-$C_6$-Alkyl oder für $C_3$-$C_6$-Alkenyl steht, dadurch gekennzeichnet, daß man den α,ω-Aminoalkohol der Formel

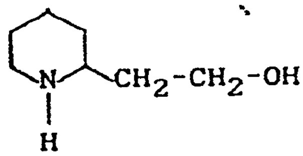

(IIa)

mit dem Carbonsäurechlorid der Formel

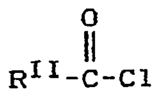

(IIIa)

wobei $R^{II}$ die oben angegebene Bedeutung besitzt,
gegebenenfalls in Gegenwart eines Säureakzeptors und gegebenenfalls unter Verwendung eines organischen Verdünnungsmittels bei Temperaturen zwischen –40 und 110°C umsetzt, die Verbindungen der Formel

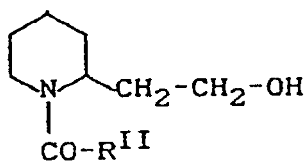

(Ib)

gegebenenfalls isoliert und gegebenenfalls weiter zum Erhalt von Verbindungen der Formel (Ia)
in welcher
$R^I$ für $COR^{11}$ steht, mit einem Carbonsäurechlorid der Formel $R^{11}$ COCl (IV) worin $R^{11}$ die obengenannte Bedeutung hat, umsetzt
oder zum Erhalt von Verbindungen der Formel (Ia),
in welcher
$R^I$ für $R^{13}$ steht,
weiter mit Alkylhalogeniden der Formel

$R^{13}$-Y (VI)

worin
$R^{13}$ die oben angegebene Bedeutung besitzt und Y für Chlor, Brom oder Iod steht,
gegebenenfalls in Gegenwart eines Säureakzeptors oder einer Base und gegebenenfalls unter Verwendung eines organischen Lösungsmittels umsetzt.

## Claims

1. Insect- and mite-repellent agents, characterized in that they contain at least one acylated α,ω-aminoalcohol derivative of the formula (I)

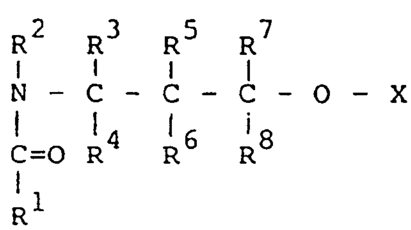

(I)

in which

X represents hydrogen, $COR^{11}$ or $R^{13}$,

R1 represents $C_1$-$C_7$-alkyl or $C_3$-$C_7$-alkenyl,

$R^2$, $R^{11}$ and $R^{13}$ are identical or different and represent $C_1$-$C_6$-alkyl,

$R^3$-$R^8$ are identical or different and represent hydrogen or $C_1$-$C_6$-alkyl, wherein $R^2$ and $R^3$, together with the atoms to which they are bonded, can form a 5- or 6-membered monocyclic ring.

2. Process for the preparation of insect- and miterepellent agents, characterized in that acylated α,ω-aminoalcohol derivatives according to formula (I) are mixed with extenders and/or surface-active agents.

3. Acylated α,ω-aminoalcohol derivatives of the formula

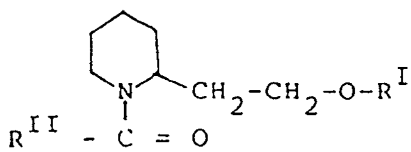

(Ia)

wherein

$R^I$ represents hydrogen, $COR^{11}$ or $R^{13}$, wherein $R^{11}$ and $R^{13}$ are identical or different and represent optionally substituted alkyl or alkenyl radicals

and

$R^{II}$ represents $C_2$-$C_6$-alkyl, or represents $C_3$-$C_6$-alkenyl.

4. Acylated α,ω-aminoalcohol derivatives of the formula (Ia) according to Claim 6, characterized in that

$R^I$ represents hydrogen or the radical $R^{13}$, wherein $R^{13}$ represents optionally substituted alkyl or alkenyl and

$R^{II}$ denotes $C_2$-$C_6$-alkyl or $C_3$-$C_6$-alkenyl.

5. Process for the preparation of acylated α,ω-aminoalcohol derivatives of the formula

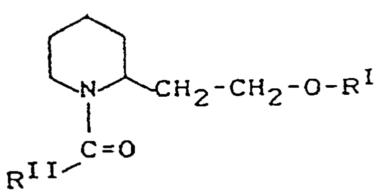

(Ia)

wherein

$R^I$ represents hydrogen, $COR^{11}$ or $R^{13}$, wherein $R^{11}$ and $R^{13}$ are identical or different and represent optionally substituted alkyl or alkenyl radicals

and

$R^{II}$ represents $C_2$-$C_6$-alkyl, or represents $C_3$-$C_6$-alkenyl,

characterized in that the α,ω-aminoalcohol of the formula

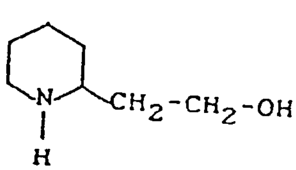

(IIa)

is reacted with the carboxylic acid chloride of the formula

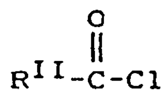

(IIIa)

wherein

$R^{II}$ has the abovementioned meaning, if appropriate in the presence of an acid acceptor and if appropriate using an organic diluent at temperatures between–40 and 100°C, the compounds of the formula

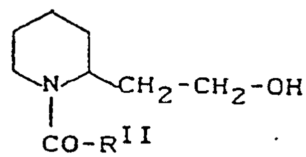

(Ib)

are isolated, if appropriate, and, if appropriate, to give compounds of the formula (Ia) in which $R^I$ represents $COR^{11}$, are further reacted with a carboxylic acid chloride of the formula $R^{11}$ COCl (IV), wherein $R^{11}$ has the abovementioned meaning, or to give compounds of the formula (Ia)
in which

$R^I$ represents $R^{13}$,

are reacted further with alkyl halides of the formula

$R^{13}$-Y (VI)

wherein

$R^{13}$ has the abovementioned meaning and

Y represents chlorine, bromine or iodine, if appropriate in the presence of an acid acceptor or a base and if appropriate using an organic solvent.

**Revendications**

1. Compositions à effet répulsif sur des insectes et des acariens, caractérisées par une teneur en au moins un dérivé d'α,ω-aminoalcool acylé de formule (I)

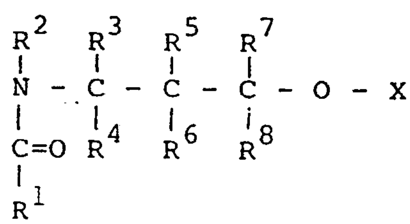

(I)

dans laquelle

X représente l'hydrogène, un groupe $COR^{11}$ ou $R^{13}$

$R^1$ est un groupe alkyle en $C_1$ à $C_7$ ou un groupe alcényle en $C_3$ à $C_7$,

$R^2$, $R^{11}$, $R^{13}$ sont identiques ou différents et représentent des groupes alkyle en $C_1$ à $C_6$,

$R^3$ à $R^8$ sont identiques ou différents et représentent l'hydrogène ou des groupes alkyle en $C_1$ à $C_6$,

$R^2$ et $R^3$ pouvant former un noyau monocyclique pentagonal ou hexagonal conjointement avec les atomes auxquels ils sont liés.

2. Procédé de préparation de compositions à effet répulsif sur des insectes et des acariens, caractérisé en ce qu'on mélange des dérivés d'α,ω-aminoalcools acylés suivant la formule (I) avec des diluants et/ou des agents tensioactifs.

3. Dérivés d'α,ω-aminoalcools acylés de formule

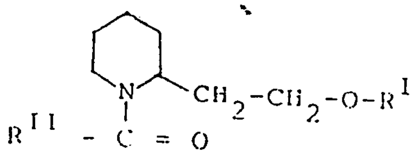

(Ia)

dans laquelle

$R^I$ représente l'hydrogène, un groupe $COR^{11}$, un groupe $R^{13}$, où $R^{11}$ et $R^{13}$ sont identiques ou différents et représentent des restes alkyle ou alcényle éventuellement substitués,

et

$R^{II}$ est un groupe alkyle en $C_2$ à $C_6$ ou alcényle en $C_3$-$C_6$.

4. Dérivés d'α,ω-aminoalcools acylés de formule (Ia) suivant la revendication 6, caractérisé en ce que

$R^I$ représente l'hydrogène ou le reste $R^{13}$, $R^{13}$ étant un groupe alkyle ou alcényle éventuellement substitué

et

$R^{II}$ est un groupe alkyle en $C_2$ à $C_6$ ou un groupe alcényle en $C_3$ à $C_6$.

5. Procédé de préparation de dérivés d'α,ω-aminoalcools acylés de formule

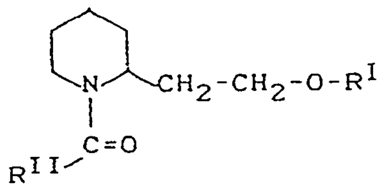

(Ia)

dans laquelle

$R^I$ représente l'hydrogène, un groupe $COR^{11}$, un groupe $R^{13}$, où $R^{11}$ et $R^{13}$ sont identiques ou différents et représentent des restes alkyle ou alcényle éventuellement substitués

et

$R^{II}$ est un groupe alkyle en $C_2$ à $C_6$ ou un groupe alcényle en $C_3$ à $C_6$,
caractérisé en ce qu'on fait réagir l'α,ω-aminoalcool de formule

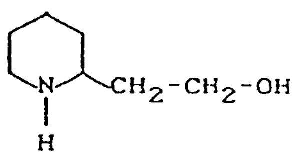

(IIa)

avec le chlorure d'acide carboxylique de formule

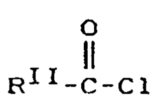

(IIIa)

où $R^{II}$ a la définition indiquée ci-dessus le cas échéant en présence d'un accepteur d'acide et avec utilisation éventuelle d'un diluant organique, à des températures comprises entre –40 et 110°C, on isole éventuellement les composés de formule

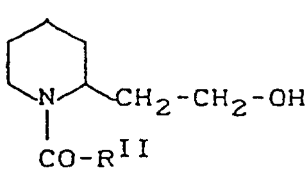

(Ib)

et, le cas échéant, pour obtenir des composés de formule (Ia)

dans laquelle

$R^I$ est un groupe $COR^{11}$, on poursuit la réaction avec un chlorure d'acide carboxylique de formule $R^{11}COCl$ (IV) dans laquelle $R^{11}$ a la définition indiquée ci-dessus, ou pour obtenir des composés de formule (Ia) dans laquelle

$R^I$ représente $R^{13}$, on poursuit la réaction avec des halogénures d'alkyle de formule

$$R^{13}\text{-}Y \quad (VI)$$

dans laquelle

$R^{13}$ a la définition indiquée ci-dessus et Y représente le chlore, le brome ou l'iode,

éventuellement en présence d'un accepteur d'acide ou d'une base et avec utilisation éventuelle d'un solvant organique.